# EUROPEAN PATENT APPLICATION

(11) **EP 1 800 711 A1**
(43) Date of publication of application: **27.06.2007**
(21) Application number: 05765697.7
(22) Date of filing: 13.07.2005
(51) Int. Cl.: A61N 1/44, A61H 33/14, A61M 35/00

(54) **COSMETIC APPARATUS**

(30) Priority: 07.09.2004 JP 2004260106; 11.05.2005 JP 2005138434
(71) Applicant: Yugen Kaisha Beauty Clinical, Aomori-shi Aomori 0300813 (JP)
(72) Inventor: Sugawara, Shigemi, Yugen Kaisha Beauty Clinical, Aomori-shi, Aomori 0300813 (JP)
(74) Representative: Texier, Christian
(86) International application number: PCT/JP2005/012923
(87) International publication number: WO 2006/027894

(57) **Abrégé**

Une électrode (2), constituée d'un groupe de fibres de carbone, est formée au bout d'un câble à haute tension (3) délivrant une haute tension d'une unité d'alimentation. L'électrode (2) est disposée dans un tube mou (4) de manière à positionner son extrémité (2a) en retrait par rapport à la partie (4a) venant en contact avec la peau du tube mou (4). L'électrode (2) est positionnée de manière à aménager un jeu entre le câble à haute tension (3) et la surface intérieure du tube mou (4) et est reliée aux moyens d'alimentation en air pour que de l'air puisse être fourni par la partie (4a) en contact avec la peau du tube mou (4). Un corps rigide cylindrique (5) est monté sur la partie périphérique du tube mou (4) de manière à couvrir cette dernière. Un matériau imprégné de liquide (6) est fixé sur la partie (4a) ouverte en contact avec la peau du tube mou (4) et les ions négatifs sont appliqués sur la peau d'un visage ou d'un bras pendant le contact de ce tube avec la peau.

## Description

La présente invention concerne un appareil cosmétique destiné à rendre moins apparentes les taches ou les rides de la peau d'un visage ou d'un bras ou à épiler.

### Art antérieur

Depuis ces dernières années, on sait empiriquement que les ions négatifs sont efficaces pour réduire les problèmes de peau tels que les taches, les contusions et les acnés sur le visage ou le bras. Ceci serait grâce au fait que les ions négatifs permettent de neutraliser et d'éliminer l'oxygène actif, une des causes des taches ou des contusions, ainsi qu'au fait que l'application des ions négatifs a pour effet d'activer l'ATP membranaire de la peau, donc d'améliorer le pouvoir de synthèse d'ATP membranaire et par conséquent la perméabilité ionique membranaire, et finalement d'obtenir un meilleur équilibre ionique intracellulaire et une activation cellulaire grâce à la migration d'un excès d'ions sodium et calcium intercellulaires vers le sang.

On propose aujourd'hui une variété de générateurs d'ions négatifs et d'appareils cosmétiques ayant de tels effets cosmétiques.

On connaît, comme un exemple de tels appareils, un appareil destiné à introduire les ions dans la partie en question d'une peau, appareil doté d'un bec à grande ouverte sur son applicateur (voir document 1 : Enregistrement du modèle d'utilité JP N° 3096200).

Un autre exemple consiste en un appareil cosmétique constitué d'un applicateur à mettre en contact avec une partie souhaitée de la peau et d'un projecteur d'un souffle contenant les ions négatifs sur ladite partie avec laquelle ledit applicateur est ou sera en contact, ledit applicateur, lui, étant constitué d'un matériau contenant de la céramique émettant de l'infrarouge lointain, un matériau contenant du germanium ou un matériau contenant de la tourmaline (voir document 2 : Publication du brevet non examiné JP N° 2004-148107).

### Présentation de l'invention

### Problèmes à résoudre par l'invention

Toutefois, un appareil cosmétique classique faisant appel à une électrode classique entrant en contact direct avec la peau a pour inconvénient de donner une sensation désagréable de piqûre à la peau et par conséquent une douleur à un utilisateur de cet appareil.

Une telle mise en contact de l'électrode avec la peau a pour inconvénient d'être peu commode, parce qu'il est nécessaire d'étudier la manière d'appliquer l'électrode et de régler avec précision son angle d'application et sa tension.

Classiquement, les ions négatifs sont produits par effluve, c'est-à-dire, par une méthode selon laquelle des électrons sont des décharges d'une électrode négative à haute tension à une électrode positive à haute tension. L'inconvénient d'une telle méthode était qu'elle produisait, outre les ions négatifs, les ions positifs, l'ozone et les oxydes d'azote qui sont nuisibles à la peau et que, par conséquent, ces substances nuisibles, non seulement réduisent l'effet esthétique, mais également constituent des causes d'un problème de peau.

A noter qu'un appareil faisant appel à l'effluve ou à l'ensemble diode émettrice d'ions et aiguille émettrice d'ions (pureté: 99,999 % d'argent) ne produit qu'une faible quantité d'ions négatifs et ne procure donc pas d'effet esthétique suffisant.

A noter également qu'il peut être nécessaire, lors d'une projection d'ions sur la peau, d'enduite celle-ci d'un sérum contenant de l'hydratant, du collagène, des vitamines etc. Dans un tel cas et avec un appareil classique, il est nécessaire de tenir le projecteur d'ions avec une main et de tapoter la peau avec un coton imbibé d'un sérum avec l'autre main et une telle pratique provoque la non-homogénéité voire l'impossibilité d'enduction du sérum sur la peau, ce qui se traduit par l'absence de l'effet esthétique ou par la baisse du rendement.

La présente invention vise donc à remédier à ces inconvénients et à proposer un appareil cosmétique indolore et ayant un effet esthétique plus prononcé. La présente invention vise à fournir un appareil cosmétique capable d'éliminer la génération des substances nuisibles à la peau.

### Moyens pour résoudre les problèmes

L'appareil cosmétique selon la présente invention est caractérisé en ce qu'il comprend une électrode, une unité d'alimentation destinée à mettre ladite électrode sous tension, un câble à haute résistance destiné à relier électriquement entre ladite électrode et ladite unité d'alimentation et un ventilateur destiné à envoyer de l'air vers ladite électrode.

Ledit câble à haute résistance peut être constitué d'une résistance polymère. D'ailleurs, ladite résistance polymère peut être du Teflon (marque déposée).

Elle peut également être une fibre de carbone.

Ledit câble et ladite électrode peuvent être disposés à l'intérieur d'un tube creux non conducteur et l'air envoyé par ledit ventilateur peut être projeté sur ladite électrode par ledit tube creux.

De plus, un jeu peut être aménagé entre ladite électrode et l'extrémité dudit tube de manière à y disposer un membre imbibé d'un liquide.

De surcroît, ledit ventilateur peut envoyer de l'air au travers d'une membrane pour la séparation des gaz qui se laisse traverser prioritairement l'oxygène. Enfin, ledit tube peut contenir de la silicone.

L'appareil cosmétique peut encore contenir un générateur d'ions positifs permettant de projeter les ions positifs sur la zone en question de ladite peau ainsi qu'un générateur d'ions permettant d'y projeter l'ozone.

### Effets de l'invention

Selon l'invention, l'appareil ne procure aucune douleur au patient, puisque son électrode n'entre jamais directement en contact avec sa peau et peut en revanche produire davantage d'ions négatifs et donc davantage d'effet esthétique. De plus, il permet de réduire la production non seulement d'ions positifs et d'ozone, mais également d'oxydes d'azote sensés être nuisibles à la peau, et donc d'éviter l'inhibition de l'effet esthétique.

### Brève description des dessins

(Fig. 1) Vue en section de la tête de projection d'ions selon le présent mode de réalisation.
(Fig. 2) Plan de dessus de l'appareil cosmétique selon le présent mode de réalisation.
(Fig. 3) Schéma électrique de l'appareil cosmétique selon le présent mode de réalisation.

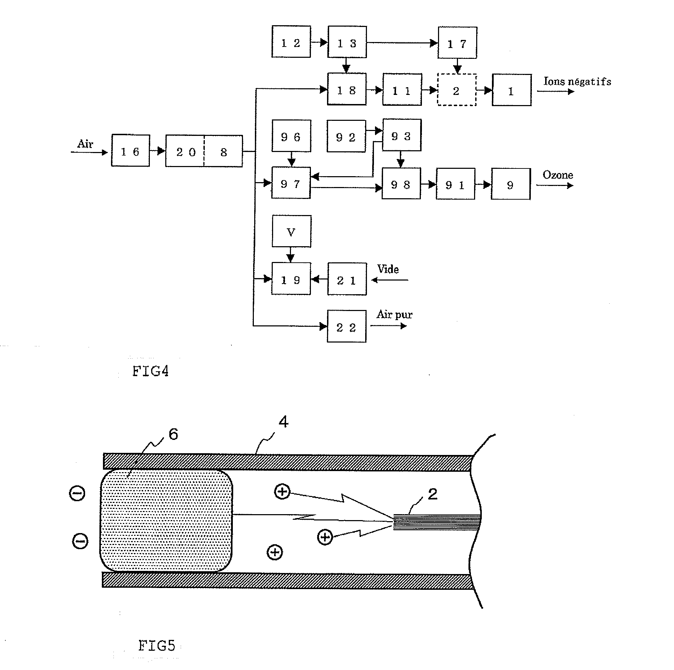

(Fig. 4) Schéma décrivant l'écoulement de l'air dans l'appareil cosmétique selon le présent mode de réalisation.
(Fig. 5) Schéma décrivant schématiquement la décharge de l'électrode selon le présent mode de réalisation.
(Fig. 6) Schéma décrivant le principe de fonctionnement du générateur d'ions positifs selon un autre mode de réalisation.

### Description détaillée

### Meilleur mode de réalisation

L'appareil cosmétique 10 selon l'un des modes de réalisation de l'invention est décrit ci-dessous en se référant aux dessins annexés.

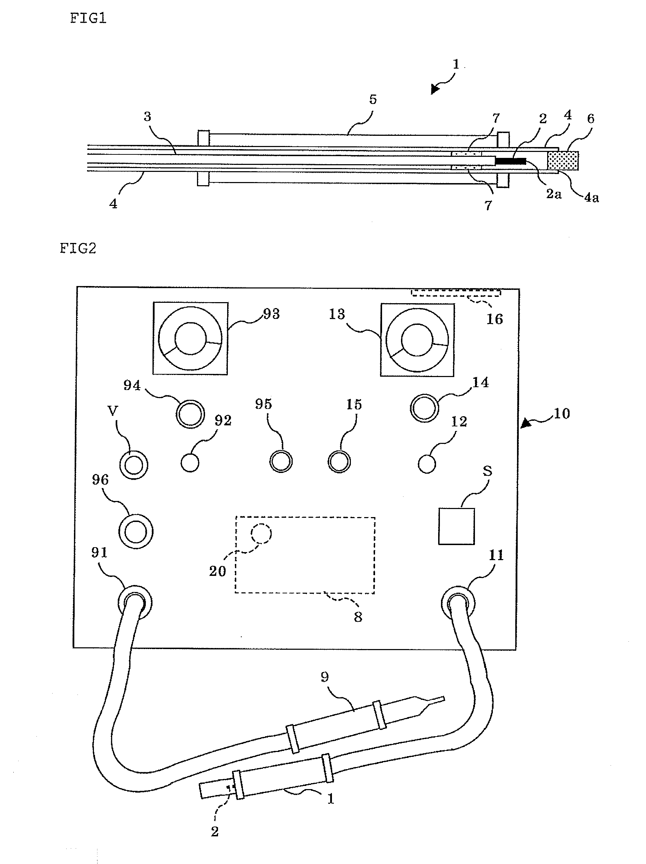

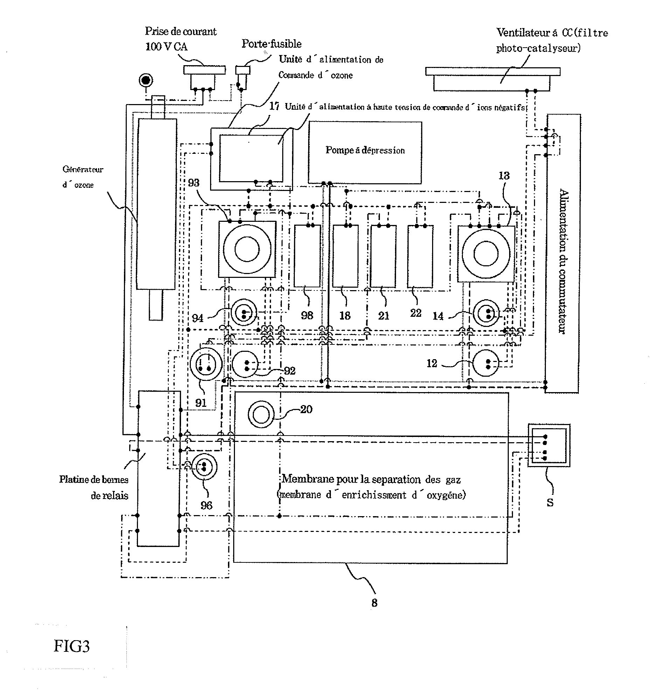

La figure 1 représente une section de la sonde d'ionisation selon le mode de réalisation de l'invention. La figure 2 représente un plan de dessus de l'appareil cosmétique selon le mode de réalisation de l'invention. La figure 3 représente un schéma électrique de l'appareil cosmétique selon le mode de réalisation de l'invention.

Les différents éléments constitutifs de l'appareil cosmétique seront d'abord décrits ci-dessous en se référant aux figures 2 et 3.

Dans les figures 2 et 3, l'appareil cosmétique 10 équipé d'une tête de projection d'ions 1 selon le présent mode de réalisation comprend: un interrupteur d'alimentation S, un bouton de commande d'aspiration V, un bouton de commande de démarrage de projection d'ions négatifs 12, une minuterie de réglage de durée de projection d'ions négatifs 13, un témoin de fonctionnement de la minuterie de réglage de durée de projection d'ions négatifs 14, une commande de réglage de débit d'air de projection d'ions négatifs 15, une unité d'alimentation haute tension de commande d'ions négatifs 17, une électrovanne de commande d'ions négatifs 18, un orifice d'aspiration d'air 20, une électrovanne d'aspiration 21, une électrovanne de projection 22, une buse de projection d'ozone 91 munie d'une tête de projection d'ozone 9, un bouton de commande de démarrage de projection d'ozone 92, une minuterie de réglage de durée de projection d'ozone 93, un témoin de fonctionnement de la minuterie de réglage de durée de projection d'ozone 94, une commande de réglage de débit d'air de projection d'ozone 95, une commande de réglage de teneur en ozone débit d'ozone 96 et une électrovanne de commande d'ozone 98.

L'interrupteur d'alimentation S constitue un interrupteur principal d'alimentation de l'appareil cosmétique 10 qui, dès son fermeture, le relie électriquement au secteur à C.A., permettant ainsi de mettre ses éléments constitutifs sous tension.

Le bouton de commande, d'aspiration 91 est un bouton assurant la commande de l'aspiration. Ce bouton de commande d'aspiration 91, lorsqu'il est activé, a pour effet de mettre l'électrovanne d'aspiration 21 sous tension et par conséquent de faire démarrer et d'arrêter l'aspiration au niveau de la tête de projection d'ozone 9.

La tête de projection d'ozone 9 sert à projeter l'ozone et à l'extrémité de laquelle est fixée une capsule à nez pointu résistante à l'acide et aux agents chimiques. Ce nez pointu permet de projeter avec précision l'ozone sur une tache de la peau.

Le tube mou 4 pour ions négatifs est doté sur son extrémité d'une tête de projection d'ions négatifs 1 et sert à alimenter cette dernière. Ce tube 4 peut être fait en silicone par exemple. A noter que l'extrémité de ce tube mou 4 est en saillie par rapport à l'électrode 2, ce qui permet d'aménager un jeu entre l'électrode 2 et l'extrémité du tube mou 4 pour y disposer et maintenir un coton ou tout autre matériau perméable et non conducteur, et par conséquent d'assurer la décharge par l'électrode avec ledit coton imbibé d'une solution cosmétique.

Le bouton de commande de démarrage 12 sert à assurer la commande de l'alimentation de l'électrovanne de commande d'ions négatifs 18 par l'unité d'alimentation haute tension de commande d'ions négatifs 17 afin de faire démarrer et d'arrêter la projection d'ions négatifs par la tête 1.

La minuterie de réglage de durée de projection d'ions négatifs 13 sert à régler la durée de projection d'ions négatifs. Cette minuterie de réglage de durée de projection d'ions négatifs 13 a donc pour fonction de mettre sous tension l'électrovanne de commande d'ions négatifs 18 par l'unité d'alimentation haute tension de commande d'ions négatifs 17 et de le maintenir pendant un temps prédéterminé, et ensuite de la mettre hors tension à l'expiration de ce temps afin d'arrêter la projection d'ions négatifs.

Le témoin de fonctionnement de la minuterie de réglage de durée de projection d'ions négatifs 14, formé d'une LED, s'allume vert lorsque le bouton de commande de démarrage 12 est activé pour faire démarrer la projection et s'éteint lorsque ce même bouton 12 est désactivé ou au bout du temps prédéterminé sur la minuterie.

La commande de réglage de débit d'air de projection d'ions négatifs 15, lui, sert à régler le débit d'ions négatifs à projeter. Cette commande de réglage de débit d'air de projection d'ions négatifs 15, constituant un SLIDAC, permet de faire varier la tension à appliquer sur l'unité d'alimentation haute tension de commande d'ions négatifs 17 par le secteur C.A., et par conséquent de faire varier le débit d'ions négatifs à projeter.

L'unité d'alimentation haute tension de commande d'ions négatifs 17 sert à délivrer une haute tension sur l'électrode 2. Dans cette description donnée à titre indicatif, cette unité d'alimentation haute tension de commande d'ions négatifs 17 est capable de délivrer une tension de 6,2 KV au maximum.

L'électrovanne de commande d'ions négatifs 18 s'ouvre et se ferme électriquement en fonction de l'opération effectuée sur le bouton de commande de démarrage de projection d'ions négatifs 12 afin d'assurer la commande de l'envoi de l'air fourni par la pompe sur le tube mou 4.

L'orifice d'aspiration d'air 20 est un orifice d'aspiration de l'air à projeter. Cet orifice d'aspiration d'air 20 est équipé d'une membrane pour la séparation des gaz 8. Comme membrane pour la séparation des gaz 8, on peut utiliser par exemple une membrane d'enrichissement d'oxygène 8 de Panasonic (PanaO₂). Cette membrane permet non seulement d'éliminer les poussières et les impuretés contenues dans l'air aspiré, mais également de réduire d'environ 10 % la quantité de l'azote contenu dans l'air. Cette propriété permet donc de réduire la production d'oxydes d'azote lors de la production des ions négatifs.

L'électrovanne d'aspiration 21 s'ouvre et se ferme en fonction de l'opération effectuée sur le bouton de commande d'aspiration 91 de manière à assurer la commande de l'aspiration par la pompe. L'électrovanne de projection 22 sert à assurer la commande la projection de l'air par la tête de projection d'ions négatifs 1.

La tête de projection d'ozone 9 sert à projeter de l'ozone. Cette tête de projection d'ozone 9 est faite en un matériau flexible tel que la silicone et permet donc d'orienter au grès la buse de la tête de projection d'ozone 9.

Le bouton de commande de démarrage de projection d'ozone 92 sert, quant à lui, à assurer l'alimentation de l'unité d'alimentation de commande d'ozone et de l'électrovanne de commande d'ozone 98.

La minuterie de réglage de durée de projection d'ozone 93 sert à régler la durée de projection d'ozone et a donc pour fonction de mettre l'unité d'alimentation de commande d'ozone hors tension à l'expiration de cette durée afin d'arrêter la projection d'ozone.

Le témoin de fonctionnement de la minuterie de réglage de durée de projection d'ozone 94, formé d'une LED, s'allume vert lorsque la minuterie de réglage de durée de projection d'ozone 93 est activée et s'éteint au bout du temps prédéterminé sur cette minuterie.

La commande de réglage de débit d'air de projection d'ozone 95, lui, sert à régler le débit d'air de projection d'ozone. Cette commande de réglage de débit d'air de projection d'ozone 95, constituant un SLIDAC, permet de faire varier la tension à appliquer sur l'électrovanne 98: Lorsqu'on fait tourner cette commande dans le sens des aiguilles d'une montre, la tension s'abaisse et le débit d'ozone diminue et lorsqu'on la fait tourner à fond dans ce même sens, la projection s'arrête.

La commande de réglage de teneur en ozone 96 sert à régler la production d'ozone (ou la teneur en ozone). Cette commande de réglage de teneur en ozone 96, constituant un SLIDAC, permet de faire varier la tension à appliquer sur l'unité d'alimentation de commande d'ozone et, par conséquent, de régler la teneur en ozone. Dans cette description donnée à titre indicatif, la teneur en ozone augmente lorsqu'on fait tourner la commande de réglage de teneur en ozone 96 dans le sens des aiguilles d'une montre et ce jusqu'à une teneur en ozone de 10 ppm au maximum.

L'électrovanne de commande d'ozone 98 s'ouvre dès l'activation du bouton de commande de démarrage de projection d'ozone 92 et reste ouverte pendant le temps prédéterminé sur la minuterie de réglage de durée de projection d'ozone 93.

Puis, la tête de projection d'ions négatifs 1 est décrite en détail en se référant à la figure 1.

Comme le montre la figure 1, la tête de projection d'ions 1 selon le présent mode de réalisation comprend une électrode 2 qui, lorsqu'on applique une haute tension, produit les ions négatifs, un câble à haute tension 3 destiné à relier électriquement entre ladite électrode 2 et l'unité d'alimentation haute tension de commande d'ions négatifs 17 (montrée sur la figure 3) et un tube mou creux en silicone 4 à l'intérieur duquel sont disposés l'électrode 2 et le câble à haute tension 3. La partie cylindrique à proximité du tube mou 4 est introduite dans un corps rigide cylindrique 5 constitué d'un tuyau en aluminium, afin d'y être protégée, et ce corps rigide cylindrique 5 sert de poignée pour l'opérateur de l'appareil.

Le câble à haute tension 3 est relié à l'électrode 2. Cette électrode 2 est formée d'un groupe d'une pluralité de fibres de carbone en tant que résistances de charge.

Cette électrode 2 est disposée dans un tube mou 4 de manière à positionner son extrémité 2a en retrait par rapport à la partie 4a venant en contact avec la peau du tube mou 4. Un jeu est aménagé entre l'extrémité 2a de l'électrode et la partie 4a venant en contact avec la peau du tube mou 4. C'est dans ce jeu qu'on peut fixer une étoffe imbibée 6 d'un sérum tel que le collagène comme le coton.

De plus, au niveau de l'électrode 2, un matériau d'espacement perméable 7 en coton est disposé entre le conducteur à haute tension 3 et l'intérieur du tube mou 4 de manière à aménager un jeu entre l'alésage du tube mou 4 et le support de l'électrode 2. Cette disposition permet le passage de l'air à travers ce matériau d'espacement perméable 7 et par conséquent la projection sur l'électrode 2.

A noter que l'électrode 2 peut être reliée au conducteur à haute tension 3 par l'intermédiaire d'un support en DELRIN (marque déposée) ou en TEFLON (marque déposée). Dans ce cas-là, un matériau d'espacement peut être disposé entre le support et l'alésage du tube mou 4.

Ce câble à haute tension 3 est constitué d'un câble à haute résistance. Ce câble à haute tension 3 peut être fait en TEFLON (marque déposée), matériau conducteur en polymère. Cette constitution rend la résistivité du câble à haute tension 3 supérieure à celle de l'électrode 2et permet donc d'appliquer une haute tension sur l'électrode 2 afin de créer une décharge.

La base du tube mou 4 est reliée (non illustrée) à la pompe de manière à pouvoir projeter l'air par la partie 4a venant en contact avec la peau. Cet air peut être fourni au travers d'une membrane pour la séparation des gaz qui se laisse traverser prioritairement par l'oxygène, par exemple une membrane d'enrichissement d'oxygène 8 de Panasonic (PanaO₂).

La procédure de projection des ions négatifs sur la peau au moyen de l'appareil cosmétique selon le mode de réalisation est décrite ci-après.
1) En premier lieu, mettre l'appareil sous tension en appuyant sur l'interrupteur d'alimentation S. Cette opération a pour résultat de mettre en marche la pompe installée dans l'appareil.
2) Puis, fixer la durée de projection d'ions négatifs en faisant tourner la roue de la minuterie de réglage de durée de projection d'ions négatifs 13.
3) Mettre l'extrémité 4a du tube 4 en contact avec le visage et appuyer sur le bouton de commande de démarrage de projection d'ions négatifs 12. Cette opération a pour effet d'activer la fonction projection d'ions négatifs et d'exciter l'électrode 2 pendant la durée prédéterminée.
4) Faire varier l'ouverture de l'électrovanne de projection 22 au moyen de la commande de réglage de débit d'air de projection d'ions négatifs 15 afin régler le débit d'air à projeter par l'extrémité 4a du tube 4 de la tête de projection 1.
5) Appuyer sur le bouton de commande d'aspiration V afin d'activer la fonction aspiration. Cette fonction n'est disponible que lorsque la projection d'ions négatifs ou d'ozone est arrêtée. Le dispositif d'aspiration sert également de tube de la tête de projection d'ozone 9.
6) Faire tourner le roue de la minuterie de réglage de durée de projection d'ozone 93 afin de régler la durée de cette fonction, mettre l'extrémité du tube de la tête de projection d'ozone 9 et appuyer sur le bouton de commande de démarrage de projection d'ozone 92. La fonction projection d'ozone sera alors activée et au bout du temps prédéterminé, elle sera désactivée.

Maintenant, le mécanisme de projection des ions négatifs par la l'électrode 2 sera décrit.

Lorsqu'une tension élevée est appliquée sur le câble à haute tension 3 par l'unité d'alimentation haute tension de commande d'ions négatifs 17, cette tension est appliquée sur l'électrode 2 sans aucune décharge de la part du câble à haute tension 3, parce que la résistivité de l'électrode 2 est inférieure à celle du câble à haute tension 3. En absence de contre-électrode par rapport à l'électrode 2, toute migration des électrons est inhibée jusqu'à ce que la valeur de la tension appliquée à l'électrode 2 dépasse un seuil et, au-delà duquel les électrons négatifs sont déchargés par l'électrode 2 d'une manière répulsive.

L'air atmosphérique contient normalement environ 30 % d'eau, ce qui signifie que les ions d'hydrogène (ions positifs) de l'eau se trouvent en suspension dans l'air atmosphérique. Les ions positifs d'autres substances existent également dans l'air atmosphérique. Il est donc possible d'avoir une décharge sans utiliser d'électrode positive physique si l'air atmosphérique est considéré comme électrode positive virtuelle. L'impédance de l'électrode 2 plus élevée que celle se trouvant entre cette électrode virtuelle et l'électrode 2 permet la décharge des électrons d'ions négatifs.

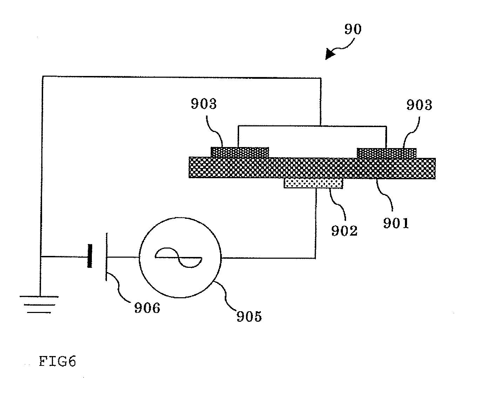

La figure 5 montre schématiquement le processus de cette décharge. Comme le montre la figure 5, l'air fourni par la pompe contient les molécules d'eau chargées positivement en suspension. Lorsqu'elles gagnent l'électrode 2, les molécules d'eau chargées électriquement subissent une décharge par l'extrémité de l'électrode 2 pour être chargées négativement. La présence du sérum dans le membre imbibé 6 fait subir à celui-ci une décharge par l'électrode, ce qui a pour effet de charger négativement ce sérum.

L'eau et le sérum négativement chargés, entraînés par l'air fourni par la pompe, traversent alors le membre imbibé 6 par l'extrémité du tube mou 4.

Pour pouvoir faire émaner les ions négatifs, il faut adapter la tension d'alimentation à l'électrode 2 et un exemple permettant une telle émanation consiste à avoir 5 V comme tension de l'unité d'alimentation à haute tension et 20 W comme résistance de charge de l'électrode 2.

A titre indicatif, nous avons mesuré la quantité d'ions négatifs projetés par l'extrémité de l'électrode 2. Cette mesure a montré que l'électrode 2 de la tête de projection 1 selon le présent mode de réalisation projette les ions négatifs avec un débit de plus de 2 millions d'ions/cm³.

Le matériel utilisé pour la mesure était un appareil de mesure d'ions de Kobe Dempa (Modèle KST-900) et les conditions de mesure étaient les suivantes:
Ions mesurés: Ions positifs et négatifs
Mobilité: Egale ou supérieure à 0,4 cm²/V.sec
Densité de charge d'espace: Différence entre le nombre d'ions positifs et le nombre d'ions négatifs, du nombre total d'ions
Environnement de mesure: Passage d'une condition atmosphérique normale vers un environnement à haute teneur par production d'ions
Plage de mesure: 5 à 999900 ions/cm³
Débit d'échantillonnage: 60 litres/min

Comme on l'a vu plus haut, le câble à haute tension 3, constitué d'un matériau dont la résistivité est supérieure à celle du matériau de l'électrode 2 et auquel une haute tension est appliquée permet à l'électrode 2 de délivrer les électrons et par conséquent la production d'une quantité plus importante d'ions négatifs. Ces ions négatifs produits en une grande quantité peuvent neutraliser ou éliminer les oxydes actifs se trouvant sur la surface d'une peau et les substances chargées positivement donc à risque toxique, permettant ainsi de réduire la stimulation de la peau et par conséquent son irritation. De plus, ces ions ont pour effet d'activer les cellules de la peau, ce qui permet de favoriser l'absorption du sérum et donc d'améliorer l'effet esthétique. A noter que le sérum se trouvant au niveau du membre imbibé 6 est ionisé lorsqu'il est projeté sur la peau, ce qui favorise l'absorption du sérum par la peau.

A noter également que l'injection de l'air dans cette électrode 2 permet une projection continue de l'air ionisé par l'extrémité du tube 4. De plus, la disposition et le maintien d'un membre 6 comme coton imbibé d'un liquide comme sérum dans le jeu aménagé entre l'électrode 2 et l'extrémité 4a du tube 4 permet une projection d'air ionisé tout en enduisant efficacement du sérum sur une partie précise de la peau et ce membre imbibé 6, non conducteur, évite toute charge directe sur la peau, ce qui veut dire que l'utilisateur n'éprouve aucune douleur ou aucun inconfort.

D'ailleurs, la production des ions positifs et de l'ozone est limitée du fait que la décharge du type précité agit sur les molécules d'eau chargées positivement, ce qui permet d'éviter toute projection fortuite de l'ozone ou des oxydes d'azote qui sont les substances nuisibles à l'effet esthétique ou ont un effet secondaire.

L'envoi de l'air au travers d'une membrane pour la séparation des gaz qui se laisse traverser prioritairement par l'oxygène permet de réduire la teneur en azote de l'air et donc d'éviter toute production des oxydes d'azote et ainsi de fournir de l'air pur exempt de poussières, d'impuretés, de bactéries et de virus.

Le fait que tube 4 est fait en silicone confère à ce tube 4 une souplesse et permet donc d'orienter au gré la buse de la tête de projection d'air ionisé, permet également un changement de tête de projection d'air ionisé et, est doux au toucher, ne donnant donc pas d'inconfort à l'utilisateur.

Le corps rigide cylindrique 5 monté sur la partie périphérique du tube mou 4 évite toute flexion du tube mou et permet donc d'améliorer la maniabilité.

La présente invention offre plusieurs possibilités de configuration: utiliser l'appareil cosmétique en tant que projecteur d'ions négatifs indépendant selon l'une des caractéristiques décrites ci-dessus, l'utiliser en tant projecteur d'ions négatifs et d'ozone et l'utiliser en tant que projecteur d'ions négatifs et positifs.

Ces possibilités permettent donc d'avoir un effet esthétique simple faisant appel aux ions négatifs, mais également un effet esthétique double faisant appel aux ions négatifs et à l'ozone ou aux ions négatifs et positifs.

Bien sûr, la présente invention n'est pas limitée au mode de réalisation décrit ci-dessus en prenant comme exemple un appareil cosmétique équipé dudit projecteur d'ions négatifs et d'un projecteur de d'ozone, mais on peut réaliser un appareil cosmétique doté dudit projecteur d'ions négatif et d'un projecteur d'ions positifs.

Maintenant, on va décrire un autre exemple de réalisation dans lequel l'appareil cosmétique équipé de projecteurs d'ions négatifs, d'ozone et d'ions positifs est appliqué à un épilateur. Dans cet exemple, il s'agit de l'appareil cosmétique selon le présent mode de réalisation équipé d'un projecteur d'ions positifs.

Dans cet exemple, les éléments constitutifs identiques à ceux du mode de réalisation ci-dessus portent les mêmes références et ne seront pas décrits en détail.

Une technique d'épilation antérieure consiste, comme le décrit la publication nationale de la version traduite (National Publication of Translated Version) JP N° 10-506554, à arracher mécaniquement ou chimiquement des poils pour faire apparaître le follicule, à l'enduire d'un photosensibilisant et à le détruire en le faisant traverser un courant électrique. Dans cette technique antérieure, un composé inactivant est injecté dans le follicule selon l'ionophorèse. Le follicule vidé après l'arrachement des poils est traversé par un courant électrique afin d'être détruit.

Cependant, selon la technique décrite ci-dessus qui consistait à arracher des pois par voie mécanique ou chimique faisant appel à un vernis, l'utilisateur éprouvait une douleur lors de l'arrachement des poils. Il éprouvait également une douleur lors d'un passage d'un courant électrique sur le follicule.

C'est l'appareil selon la présent mode de réalisation qui a résolu ce problème.

Dans ce mode de réalisation, l'appareil cosmétique 10 décrit selon le présent mode de réalisation comprend un générateur d'ions positifs tel que représenté sur la figure 6 et une unité d'alimentation à haute tension du générateur d'ions positifs et les ions positifs générés par l'électrovanne sont projetés par exemple par la tête de projection 1, etc.

Ce générateur d'ions positifs est constitué d'une électrode de décharge 902 disposée sur une surface du corps diélectrique 901 en céramique ou en mica et d'une électrode inductive 903 disposée sur l'autre face de même corps, et a pour fonction d'ioniser l'air ambiant en générant une décharge (un plasma) en appliquant une tension à C.A. 906 et une tension de polarisation à C.C. 905 entre cette électrode de décharge 902 et l'électrode inductive 903.

Le fait qu'une tension de polarisation à C.C. est appliquée sur l'électrode de décharge 902 permet à l'électrode de décharge 902 d'absorber les ions négatifs et de ne fournir que les ions positifs de manière à pouvoir projeter ceux-ci par la tête 1 ou par la tête 2 en commandant l'électrovanne.

Maintenant, la procédure d'épilation au moyen de l'appareil cosmétique décrit ci-dessus.

D'abord, on enduit une partie de la peau à épiler (par exemple l'aisselle, la jambière ou le bras) d'une solution dépilatoire. Celle-ci est une solution contenant de l'isoflavone, de la papaïne et de l'hydratant. Lorsque la peau est enduite de cette solution, la papaïney contenu, protéase, décompose les cellules des poils et des racines. L'isoflavone contenue dans cette solution, quant à elle, joue un rôle similaire à celui des estrogènes qui sont les hormones femelles, c'est-à-dire, elle agit sur les cellules et réduit la pousse des poils.

Puis, on positionne la tête de projection d'ozone 9 sur la partie à épiler et la fait projeter l'ozone. Cette opération permet de débarrasser la surface de la peau de taches et de bactéries diverses et d'inactiver les cellules de poils à épiler.

Ensuite, on positionne la tête de projection d'ions négatifs 1 sur la partie à épiler et la fait projeter les ions négatifs. Cette opération permet d'activer la peau à épiler et par conséquent d'augmenter l'ouverture des pores à poils afin de favoriser l'absorption de la solution dépilatoire etc. par la peau. En effet, la projection d'ions négatifs a pour effet non seulement d'activer l'ATPase des membranes cellulaires, et par conséquent, de favoriser la synthèse d'ATP, mais également d'accroître la perméabilité aux ions des membranes cellulaires et donc de favoriser la migration des ions de sodium et de potassium en superflu se trouvant dans les cellules vers le sang, ce qui autorise un meilleur équilibre ionique dans les cellules, une activation de ces dernières et une favorisation de l'absorption de la solution dépilatoire.

Durant cette opération, il est possible de fixer un membre imbibé tel qu'un coton imbibé d'une solution dépilatoire dans un jeu aménagé à l'extrémité du tube.

Finalement, on bascule l'électrovanne et on fait projeter les ions positifs par la tête de projection 1 d'ions négatifs afin de les projeter et les injecter dans la peau à épiler. Les ions positifs injectés au niveau des cellules de racine de poils et de follicule ont pour fonction de les inactiver.

Leur inactivation par les ions positifs injectés a pour résultat de détruire ces cellules et de faciliter leur arrachement. De plus, leur inactivation par la solution dépilatoire et les ions positifs réduit la pousse des poils.

Alors que les projecteurs distincts d'ions négatifs et positifs ont été décrits dans le mode de réalisation ci-dessus, il est également possible de faire de manière à ce qu'une seule tête 1 serve à les projeter en même temps.

### Liste des symboles

S ... Interrupteur d'alimentation,
V ... Bouton de commande d'aspiration,
1 ... Tête de projection d'ions négatifs,
2 ... Electrode,
3 ... Câble à haute tension,
4 ... Tube mou,
4a ... Partie venant en contact avec la peau,
5 ... Corps rigide cylindrique,
6 ... Membre imbibé,
7 ... Matériau d'espacement perméable,
8 ... Membrane d'enrichissement d'oxygène,
9 ... Tête de projection d'ozone,
10 ... Appareil cosmétique,
12 ... Bouton de commande de démarrage de projection d'ions négatifs,
13 ... Minuterie de réglage de durée de projection d'ions négatifs,
14 ... Témoin de fonctionnement de la minuterie de réglage de durée de projection d'ions négatifs,
15 ... Commande de réglage de débit d'air de projection d'ions négatifs,
20 ... Orifice d'aspiration d'air,
92 ... Bouton de commande de démarrage de projection d'ozone,
93 ... Minuterie de réglage de durée de projection d'ozone,
94 ... Témoin de fonctionnement de la minuterie de réglage de durée de projection d'ozone,
95 ... Commande de réglage de débit d'air de projection d'ozone, 96 ... Commande de réglage de teneur en ozone débit d'ozone.

## Revendications

1. Appareil cosmétique destiné à conférer un effet esthétique sur la peau, comprenant:
une électrode (2),
une unité d'alimentation (17) destinée à mettre ladite électrode sous tension,
un câble (3) destiné à relier électriquement ladite électrode et ladite unité d'alimentation, ledit câble ayant une résistance plus haute que ladite électrode,
un ventilateur destiné à envoyer de l'air vers ladite électrode, et
un tube (4) creux non conducteur destiné à contenir ledit câble et ladite électrode et à laisser passer l'air fourni par ledit ventilateur,
**caractérisé en qu'**il est constitué de telle manière qu'un jeu soit aménagé entre ladite électrode et l'extrémité dudit tube de manière à y disposer un membre perméable aux gaz et imbibé d'un liquide qui, après être imbibé d'une solution esthétique, soit relié à ladite électrode pour pouvoir effectuer une décharge.

2. Appareil cosmétique selon la revendication 1, dans lequel ladite résistance est en polymère, préférentiellement en Teflon (marque déposée).

3. Appareil cosmétique selon la revendication 1, dans lequel ladite électrode est en fibres de carbone.

4. Appareil cosmétique selon la revendication 1, dans lequel ledit tube contient de la silicone.

5. Appareil cosmétique selon la revendication 1, dans lequel ledit ventilateur envoie de l'air au travers d'une membrane pour la séparation des gaz qui se laisse traverser prioritairement par l'oxygène.

6. Appareil cosmétique selon la revendication 1, comprenant de plus:
un générateur d'ions positifs destiné à les générer et les projeter sur une partie en question de ladite peau, et
un générateur d'ozone destiné à le générer et le projeter sur la partie en question de ladite peau.
